# EUROPEAN PATENT APPLICATION

(11) **EP 4 338 730 A1**
(43) Date of publication of application: **20.03.2024**
(21) Application number: 22806851.6
(22) Date of filing: 13.05.2022
(51) Int. Cl.: A61K 9/48, A61K 9/20, C07D 471/06, C07B 59/00, A61K 31/506, A61P 35/00, A61P 35/02

(54) **PHARMACEUTICAL COMPOSITION**

(30) Priority: 14.05.2021 CN 202110532480
(71) Applicant: Nanjing Chuangte Pharmaceutical Technology Co., Ltd., Nanjing, Jiangsu 210009 (CN)
(72) Inventor: ZHU, Yongqiang, Nanjing, Jiangsu 210009 (CN); TANG, Yun, Nanjing, Jiangsu 210009 (CN); LIU, Shuang, Nanjing, Jiangsu 210009 (CN); YUAN, Xiaoyuan, Nanjing, Jiangsu 210009 (CN); DONG, Zexi, Nanjing, Jiangsu 210009 (CN); LIU, Xuan, Nanjing, Jiangsu 210009 (CN)
(74) Representative: Engelhard, Markus
(86) International application number: PCT/CN2022/092583
(87) International publication number: WO 2022/237889

(57) **Abstract**

The present discloses a pharmaceutical formulations containing N-(5-((4-(5,6-dihydro-4H-pyrrolo[3,2,1-ij]quinolin-1-yl)pyrimidin-2-yl)amino)-4-met hoxy-2-(methyl(2-(methyl(methyl-d₃)amino)ethyl)amino)phenyl)acetamide or a pharmaceutically acceptable salt thereof as the active ingredient. The formulations are combined with at least one pharmaceutically acceptable excipient, and it is formulated into orally administrable preparations suitable for clinical use. The pharmaceutical formulations can ensure that the active ingredient have good stability and dissolution effect. The formulated samples exhibit favorable dissolution uniformity under different dissolution media, while feature a simple production process that yields higher.

## Description

### TECHNICAL FIELD

The present invention falls within the realm of pharmaceutical formulations, and relates to pharmaceutical formulations and the preparation method thereof.

### BACKGROUND ART

The Epidermal Growth Factor Receptor (EGFR), a tyrosine kinase receptor responding to epidermal growth factor (EGF) by engaging in cell proliferation and signal transduction, is a component of ErbB receptor family. Located on the cell membrane, EGFR undergoes dimerization upon binding with its ligands (EGF and transforming growth factor TGFα, etc.). This dimerization event subsequently triggers the activation of EGFR's intracellular kinase pathways. These pathways are associated with processes such as tumor cell proliferation, angiogenesis, tumor invasion, metastasis, and inhibition of cellular apoptosis.

Members of the EGFR family play crucial roles in normal development, but are often overexpressed and dysregulated in human tumors. In 50%-80% of NSCLC patients, overexpression of the EGFR triggers carcinogenesis. Studies have shown that inhibiting EGFR can interrupt cell signaling, consequently suppressing cell growth and proliferation. EGFR has consistently represented a prominent target for tumor treatment.

The evolution of EGFR TKI drugs has progressed from the first generation to the third. Distinct EGFR TKIs exhibit slight variations in pharmacological mechanisms. First generation EGFR TKIs (gefitinib, erlotinib and icotinib) reversibly bind to EGFR. Second generation EGFR TKIs (e.g., afatinib, dacomitinib) are multi-target small molecule agents, capable of forming irreversible covalent bonds with the cysteine residue at position 797 of EGFR. Additionally, they display inhibitory activity against other members of the ErbB family (e.g., wild-type EGFR, ErbB-2, ErbB-4). The third-generation EGFR TKIs (epitomized by osimertinib) are profoundly selective small molecule inhibitors targeting the T790M mutation. Despite the continuous expansion of the EGFR TKI drug repertoire, acquired drug resistance remains a challenge. A pressing clinical demand exists for novel compounds, particularly those incorporating innovative frameworks, to address challenges such as drug resistance and suboptimal selectivity.

The compound N-(5-((4-(5,6-dihydro-4H-pyrrolo[3,2,1-ij]quinoline-1-yl) pyrimidin-2-yl)amino)-4-methoxy-2-(methyl(2-(methyl(methyl-d₃)amino)ethyl)amino )phenyl)acetamide is a third-generation small molecule EGFR TKI agent. This compound notably demonstrates heightened selectivity in inhibiting L858R activating mutations, Exon19 deletion mutations, and T790M resistance mutations. It finds application in treating disorders mediated by EGFR or arising from activating mutations or resistance mutations of EGFR. This compound, along with the pharmaceutically acceptable salts thereof, is disclosed in patent applications WO2018050108 and WO2019174623. However, the compound's poor hygroscopicity poses challenges in tablet formulation.

### SUMMARY OF INVENTION

The present invention provides pharmaceutical formulations containing N-(5-((4-(5,6-dihydro-4H-pyrrolo[3,2,1-ij]quinolin-1-yl)pyrimidin-2-yl)amino)-4-met hoxy-2-(methyl(2-(methyl(methyl-d3)amino)ethyl)amino)phenyl)acetamide or a pharmaceutically acceptable salt thereof.

The pharmaceutical formulations encompass N-(5-((4-(5,6-dihydro-4H-pyrrolo [3,2,1-ij]quinolin-1-yl)pyrimidin-2-yl)amino)-4-methoxy-2-(methyl(2-(methyl(methyl -d₃)amino)ethyl)amino)phenyl)acetamide or a pharmaceutically acceptable salt thereof as the active ingredient. The formulations are combined with at least one pharmaceutically acceptable excipient, and it is formulated into orally administrable preparations suitable for clinical use.

In the pharmaceutical composition of the present invention, the active ingredient is N-(5-((4-(5,6-dihydro-4H-pyrrolo[3,2,1-ij]quinolin-1-yl)pyrimidin-2-yl)amino) -4-methoxy-2-(methyl(2-(methyl(methyl-d3)amino)ethyl)amino)phenyl)acetamide or a pharmaceutically acceptable salt thereof, wherein the salt may be in crystalline form.

Furthermore, in the formulations of the present invention, the active ingredient is N-(5-((4-(5,6-dihydro-4H-pyrrolo[3,2,1-ij]quinolin-1-yl)pyrimidin-2-yl)amino)-4-met hoxy-2-(methyl(2-(methyl(methyl-d3)amino)ethyl)amino)phenyl)acetamide methanesulfonate.

Furthermore, in the formulations of the present invention, the oral formulation is capsule.

Furthermore, in the formulations of the present invention, the excipients encompass one or more types of filler, such as microcrystalline cellulose, mannitol, one or more types of lactose; preferably, the filler is one or a combination of microcrystalline cellulose and mannitol.

Furthermore, in the formulations of the present invention, the excipients encompass one or more types of disintegrants, selected from sodium starch glycolate, croscarmellose sodium, low-substituted hydroxypropyl cellulose; preferably, the disintegrant is low-substituted hydroxypropyl cellulose. The formulations of the present invention can also be devoid of disintegrant.

Furthermore, in the formulations of the present invention, the excipients include one or more types of lubricants, selected from talc, magnesium stearate, silica, or one or more types of sodium stearyl fumarate; preferably, the lubricant is one or a combination of sodium stearyl fumarate, magnesium stearate or talc.

According to the formulations of the present invention, the pharmaceutical formulations, formulated into a clinically acceptable capsule, includes the following components, or consists of the following components:

| | |
|---|---|
| Active ingredient | 1-40% |
| Filler | 1-80% |
| Disintegrant | 0-10% |
| Lubricant | 1-5%; |

The percentages of each component are in accordance with weight percentages, and the sum of the percentages of all components totals 100%.

According to the formulations of the present invention, the pharmaceutical formulations, formulated into a clinically acceptable capsule, includes the following components, or consists of the following components:

| | |
|---|---|
| Active ingredient | 20-38% |
| Filler | 60-75% |
| Lubricant | 1-5%; |

The percentages of each component are in accordance with weight percentages, and the sum of the percentages of all components totals 100%. The inventors have discovered that capsules formulated with these components not only exhibit simple excipients compositions and low cost, but also possess the capability to efficiently address the dissolution delay associated with the capsule shell. This notably enhances the dissolution rate of the capsule formulation, particularly within a short timeframe.

Preferably, the pharmaceutical formulation, formulated into a clinically acceptable capsule, includes the following components, or consists of the following components:

| | |
|---|---|
| Active ingredient | 23.60-37.82% |
| Microcrystalline cellulose | 60.60-74.72% |
| Magnesium stearate | 0.50-0.52% |
| Talc | 1.08-1.12%; |

The percentages of each component are in accordance with weight percentages, and the sum of the percentages of all components totals 100%.

In one specific embodiment, the pharmaceutical formulation, formulated into a clinically acceptable capsule, consists of the following components:

| | |
|---|---|
| Active ingredient | 8.43% |
| Mannitol | 25.36% |
| Microcrystalline cellulose | 59.21% |
| Low-substituted hydroxypropyl cellulose | 5.00% |
| Magnesium stearate | 0.50% |
| Talc | 1.50%; |

In one specific embodiment, the pharmaceutical formulation, formulated into a clinically acceptable capsule, consists of the following components:

| | |
|---|---|
| Active ingredient | 23.65% |
| Microcrystalline cellulose | 69.35% |
| Low-substituted hydroxypropyl cellulose | 5.00% |
| Magnesium stearate | 0.50% |
| Talc | 1.50%. |

In one specific embodiment, the pharmaceutical formulation, formulated into a clinically acceptable capsule, consists of the following components:

| | |
|---|---|
| Active ingredient | 23.64% |
| Microcrystalline cellulose | 74.72% |
| Magnesium stearate | 0.52% |
| Talc | 1.12%. |

In one specific embodiment, the pharmaceutical formulation, formulated into a clinically acceptable capsule, consists of the following components:

| | |
|---|---|
| Active ingredient | 37.82% |
| Microcrystalline cellulose | 60.60% |
| Magnesium stearate | 0.50% |
| Talc | 1.08%. |

The present invention also provides a method for preparing the formulations:
(1) Mixing: After sieving the raw materials, take the active ingredient and a portion of the filler as specified in the pharmaceutical formulations and mix it with at 10-20 rpm for 4-10 min; add the remaining filler and continue mixing at 10-20 rpm for 4-10 min; add the lubricant and mix at 10-20 rpm for 8-15 min;
(2) Capsule filling: At the temperature of 15°C to 25°C and relative humidity between 45% and 65%, calculate the actual filling quantity by considering the intermediate content and theoretical filling content, and proceed with capsule filling process.

In certain embodiments, the preparation of the present invention may involve using 40% to 60% of the filler of the pharmaceutical formulations in step (1), and in specific embodiments, half the weight of the filler is required in the pharmaceutical formulation.

The preparation method of the present invention, unless otherwise specified, can be carried out according to conventional methods in the art.

The active ingredient content in the present invention, unless otherwise specified, is "N-(5-((4-(5,6-dihydro-4H-pyrrolo[3,2,1-ij]quinolin-1-yl)pyrimidin-2-yl)amino) -4-methoxy-2-(methyl(2-(methyl(methyl-d3)amino)ethyl)amino)phenyl)acetamide methanesulfonate".

The "%" in the ratios of the present invention, unless otherwise specified, refers to the weight percentage, which specifically refers to the weight percentage of one component in the total components. For example, in the context of 100 g of total components, 10% signifies 10 g of the component.

The capsule formulation in the present invention is not limited by specific capsule shell types. The capsule shell type does not affect the effectiveness of this patent.

Beneficial Effects:(1) The oral formulation containing the compound of N-(5-((4-(5,6-dihydro-4H-pyrrolo[3,2,1-ij]quinolin-1-yl)pyrimidin-2-yl)amino)-4-met hoxy-2-(methyl(2-(methyl(methyl-d3)amino)ethyl)amino)phenyl)acetamide methanesulfonate, as provided by the present invention, ensures the good stability and dissolution performance of N-(5-((4-(5,6-dihydro-4H-pyrrolo[3,2,1-ij]quinolin-1-yl) pyrimidin-2-yl)amino)-4-methoxy-2-(methyl(2-(methyl(methyl-d3)amino)ethyl)amin o)phenyl)acetamide methanesulfonate. The formulated samples exhibit favorable dissolution uniformity under different dissolution media.

(2) The capsules formulated according to the present invention can rapidly disintegrate, disperse, dissolve and be absorbed. Within the context of the present invention, each dosage unit is ensured to have high drug loading with minimal excipients varieties, thereby reducing the interactions between excipients and the drug. These capsules can also effectively overcome the dissolution delay caused by the capsule shell and significantly improve the dissolution rate of the capsule, particularly within a short timeframe.

(3) The capsules prepared according to the present invention, while not containing disintegrants, feature a simple production process that yields higher, up to 84% or more, ensuring a smoother formulation process and further enhancing yields

### DESCRIPTION OF THE PREFERRED EMBODIMENTS

The following detailed examples further illustrate the present invention in conjunction with common technical knowledge and conventional means in the art. These examples are only partial preferred embodiments of the present invention and should not be construed as limitations thereof. Those skilled in the art can make several improvements within the scope of the present invention, and these improvements should also be considered as the protection scope of the present invention.

### Example 1: (Capsule 1)

A pharmaceutical formulation containing N-(5-((4-(5,6-dihydro-4H-pyrrolo [3,2,1-ij]quinolin-1-yl)pyrimidin-2-yl)amino)-4-methoxy-2-(methyl(2-(methyl(methyl -d₃)amino)ethyl)amino)phenyl)acetamide methanesulfonate, with the pharmaceutical formulation comprising 23.6 mg of active ingredient (calculated as C₃₁H₃₆D₃N₇O₅S), 71.0 mg of mannitol (100SD), 165.8 mg of microcrystalline cellulose (112), 14 mg of low-substituted hydroxypropyl cellulose (LH-21), 1.4 mg of magnesium stearate, and 4.2 mg of talc.

The capsule containing the above components is formulated by the following method:
(1) Pretreatment of raw materials and excipients as shown in Table 1;

**Table 1**

| Materials | Processing method |
|---|---|
| Active ingredient | Sieve, 40 mesh |
| Mannitol (100SD) | Sieve, 40 mesh |
| Microcrystalline cellulose (112) | Sieve, 40 mesh |
| Low-substituted hydroxypropyl cellulose (LH-21) | Sieve, 40 mesh |
| Magnesium stearate | Sieve, 40 mesh |
| Talc | Sieve, 60 mesh |

(2) Weighing: Weigh the raw materials and excipients according to the pharmaceutical formulation;
(3) Mixing: Successively mix the active ingredient, mannitol 100SD, microcrystalline cellulose 112, low-substituted hydroxypropyl cellulose, magnesium stearate, and microcrystalline cellulose (added in two equal portions) in a mixer. Alternate mix using a granulator and a hopper mixer to ensure the materials are thoroughly and evenly mixed;
(4) Granulation:1) Primary granulation: granulate with dry granulator, with upper screen of 5 mm and lower screen of 1.2 mm;2) Primary sieving: Pass the granules through an 80-mesh screen. The granules retained on 80-mesh sieve are recorded as granule①, while the granules passing through 80-mesh sieve are subjected to secondary granulation;3) Secondary granulation: Granulate using dry-type granulator, with lower screen of 1.2 mm; 4) Secondary sieving: Pass the granules through an 80-mesh sieve, with particles larger than 80 mesh identified as Granule②;
(5) Final mixing: Thoroughly and uniformly mix Granule①, Granule②, and talc in a hopper mixer at 15 rpm for 10 minutes;
(6) Capsule filling: Utilize #1 capsule shells for capsule filling. Calculate the actual filling quantity based on the intermediate content and theoretical filling content. Pilot batch: 22,000 capsules; total yield: 58.5%;
(7) Packaging: Employ PA/AL/PVC COLD-formed Foil for Solid Pharmaceutical Packaging + pharmaceutical AL foil for packaging the filled capsules.

### Example 2: (Capsule 2)

A pharmaceutical formulation containing N-(5-((4-(5,6-dihydro-4H-pyrrolo [3,2,1-ij]quinolin-1-yl)pyrimidin-2-yl)amino)-4-methoxy-2-(methyl(2-(methyl(methyl -d3)amino)ethyl)amino)phenyl)acetamide methanesulfonate, with the pharmaceutical formulation comprising 94.6 mg of active ingredient (calculated as C31H36D3N7O5S), 277.4 mg of microcrystalline cellulose (112), 20 mg of low-substituted hydroxypropyl cellulose (LH-21), 2.0 mg of magnesium stearate, and 6.0 mg of talc.

The capsule containing the above components is formulated by the following method:
(1) Pretreatment of raw materials and excipients as shown in Table 2;

**Table 2**

| Materials | Processing method |
|---|---|
| Active ingredient | Sieve, 40 mesh |
| Microcrystalline cellulose (PH112) | Sieve, 40 mesh |
| Low-substituted hydroxypropyl cellulose (LH-21) | Sieve, 40 mesh |
| Magnesium stearate | Sieve, 40 mesh |
| Talc | Sieve, 60 mesh |

(2) Weighing: Weigh the raw materials and excipients according to the pharmaceutical formulation;
(3) Mixing: Successively mix the active ingredient, microcrystalline cellulose 112 (added in two equal portions), low-substituted hydroxypropyl cellulose, magnesium stearate in a mixer. Alternate mix using a granulator and a hopper mixer to ensure the materials are thoroughly and evenly mixed;
(4) Granulation: 1) Primary granulation: Granulate with dry granulator, with upper screen of 5 mm and lower screen of 1.2 mm;2) Primary sieving: Pass the granules through an 80-mesh sieve. The granules retained on 80-mesh sieve are recorded as granule①, while the granules passing through 80-mesh sieve are subjected to secondary granulation; 3) Secondary granulation: Granulate using dry granulator, with lower screen of 1.2 mm;4) Secondary sieving: Pass the granules through an 80-mesh screen, with particles larger than 80 mesh identified as Granule ②;
(5) Final mixing: Thoroughly and uniformly mix Granule①, Granule②, and talc in a hopper mixer at 15 rpm for 10 minutes;
(6) Capsule filling: Utilize #0 capsule shells for capsule filling. Calculate the actual filling quantity based on the intermediate content and theoretical filling content. Pilot batch: 22,000 capsules; total yield: 54.6%;
(7) Packaging: Employ PA/AL/PVC COLD-formed Foil for Solid Pharmaceutical Packaging + pharmaceutical AL foil for packaging the filled capsules.

### Example 3: (Capsule 3)

A pharmaceutical formulation containing N-(5-((4-(5,6-dihydro-4H-pyrrolo[3,2,1-ij] quinolin-1-yl)pyrimidin-2-yl)amino)-4-methoxy-2-(methyl(2-(methyl(methyl-d₃)amin o)ethyl)amino)phenyl)acetamide methanesulfonate, with the pharmaceutical formulation comprising 23.635 mg of active ingredient (calculated as C₃₁H₃₆D₃N₇O₅S), 74.715 mg of microcrystalline cellulose (112), 0.52 mg of magnesium stearate, and 1.12 mg of talc.

The capsule containing the above components is formulated by the following method:
(1) Pretreatment of raw materials and excipients: Sieve the raw materials and excipients through a 40-mesh sieve;
(2) Weighing: Weigh the raw materials and excipients according to the predetermined amounts in the pharmaceutical formulation;
(3) Mixing: 1) Pre-mix I: Add the active ingredient and half of the microcrystalline cellulose (112) into a hopper mixer. Mix at a speed of 15 rpm for 5 minutes; 2) Pre-mix II: Add the remaining microcrystalline cellulose to the hopper mixer after Pre-mix I. Mix at a speed of 15 rpm for 5 minutes;3) Pre-mix III: Add magnesium stearate and talc to the hopper mixer after Pre-mix II. Mix at a speed of 15 rpm for 10 minutes;
(4) Capsule Filling: Under the conditions of 15°C to 25°C and relative humidity between 45% and 65%, calculate the actual filling quantity based on the intermediate content and theoretical filling content. Choose #4 capsule shells for capsule filling. Pilot batch: 50,000 capsules; total yield: 84.6%;
(5) Packaging: Employ PVC/PVDC COLD-formed Foil for Solid Pharmaceutical Packaging + pharmaceutical AL foil for packaging the filled capsules.

### Example 4: (Capsule 4)

A pharmaceutical formulation containing N-(5-((4-(5,6-dihydro-4H-pyrrolo [3,2,1-ij]quinolin-1-yl)pyrimidin-2-yl)amino)-4-methoxy-2-(methyl(2-(methyl(methyl -d₃)amino)ethyl)amino)phenyl)acetamide methanesulfonate, with the pharmaceutical formulation comprising 94.54 mg of active ingredient (calculated as C₃₁H₃₆D₃N₇O₅S), 151.5 mg of microcrystalline cellulose (112), 1.26 mg of magnesium stearate, and 2.7 mg of talc.

The capsule containing the above components is formulated by the following method:
(1) Pretreatment of raw materials and excipients: Sieve the raw materials and excipients through a 40-mesh sieve;
(2) Weighing: Weigh the raw materials and excipients according to the predetermined amounts in the pharmaceutical formulation;
(3) Mixing: 1) Pre-mix I: Add the active ingredient and half of the microcrystalline cellulose (112) into a hopper mixer. Mix at a speed of 15 rpm for 5 minutes; 2) Pre-mix II: Add the remaining microcrystalline cellulose to the hopper mixer after Pre-mix I. Mix at a speed of 15 rpm for 5 minutes; 3) Pre-mix III: Add magnesium stearate and talc to the hopper mixer after Pre-mix II. Mix at a speed of 15 rpm for 10 minutes.
(4) Capsule Filling: Under the conditions of 15°C to 25°C and relative humidity between 45% and 65%, calculate the actual filling quantity based on the intermediate content and theoretical filling content. Choose #1 capsule shells for capsule filling. Pilot batch: 100,000 capsules; total yield: 93.6%;
(5) Packaging: Employ PVC/PVDC COLD-formed Foil for Solid Pharmaceutical Packaging + pharmaceutical;

### DISSOLUTION TESTING AND RESULTS ANALYSIS

Dissolution testing was performed on the products prepared from Example 3 and Example 4 using various media, including pH 1.3 hydrochloric acid solution and pH 6.8 phosphate buffer solution. The outcomes are presented in Table 3 and Table 4. The products formulated by this invention displayed favorable dissolution profiles across diverse media.

**Table 3 Dissolution Profiles of 20 mg Specification (Example 3) in Two Media**

| Time (min) | pH1.3 Hydrochloric Acid Solution | pH6.8 Phosphate Buffer Solution |
|---|---|---|
| | Average Dissolution (%) | |
| 5 | 97.5 | 11.4 |
| 10 | 98.8 | 24.9 |
| 15 | 98.9 | 31.4 |

**Table 4 Dissolution Profiles of 80 mg Specification (Example 4) in Two Media**

| Time (min) | pH1.3 Hydrochloric Acid Solution | pH6.8 Phosphate Buffer Solution |
|---|---|---|
| | Average Dissolution (%) | |
| 5 | 100.8 | 14.9 |
| 10 | 105.1 | 26.3 |
| 15 | 105.3 | 32.5 |

Dissolution testing was conducted on the products prepared from Example 3 and Example 4 using different media (pH 1.3 hydrochloric acid solution and pH 6.8 phosphate buffer solution), as indicated in Table 3 and Table 4. The results demonstrate that the products prepared by the present invention exhibit favorable dissolution characteristics across diverse media, effectively overcoming the delay in dissolution attributed to the capsule shell and significantly enhancing the dissolution rate of the capsule formulation.

Furthermore, experimental validation has confirmed that this patent does not impose specific requirements on the capsule shell type. The capsule shell type does not affect the effectiveness of this patent.

Using the same method, dissolution testing was conducted on the products prepared from Example 1 and Example 2. The cumulative dissolution after 15 minutes in pH 1.3 hydrochloric acid solution ranged from 85% to 98%, and in pH 6.8 phosphate buffer solution, it ranged from 45% to 59%.

### STABILITY TESTING AND RESULT ANALYSIS

Accelerated and long-term stability tests were performed on products derived from both Example 1 and Example 2. These products were sealed in double aluminum blister packaging and subjected to two environmental conditions: one at a temperature of 40±2°C with a relative humidity of 75±5%, and the other at 30±2°C with a relative humidity of 65±5%. The accelerated tests spanned a duration of 6 months, and the long-term tests extended for the same period. The results of these extensive investigations have been summarized in Table 5, Table 6, Table 7, and Table 8. It is worth noting that throughout the entire 6-month period of both accelerated and long-term testing, all parameters consistently remained within acceptable limits. There were no notable or significant deviations observed, thus affirming the product's exceptional stability under the specified packaging conditions.

**Table 5 Accelerated Stability Test Results for Capsule Formulation (Example 1)**

| Evaluation Parameter | | Requirements | Starting time | 1 Month | 2 Months | 3 Months | 6 Months |
|---|---|---|---|---|---|---|---|
| Appearance | | Content should be pale yellow to yellow powder or granules | Yellow Granules | Yellow Granules | Yellow Granules | Yellow Granules | Yellow Granules |
| Related Substances (%) | Individual Impurities | should be <0.5% | 0.04 | 0.04 | 0.04 | 0.04 | 0.04 |
| | Total Impurities | should be ≤1.5% | 0.20 | 0.34 | 0.33 | 0.36 | 0.58 |
| Dissolution (%) | | Should be ≥80% of labeled amount | 101.7 | 92.3 | 95.5 | 98.2 | 96.2 |
| Content (%) | | C₃₀H₃₂D₃N₇O₂ should be 90% ∼ 110% of labeled amount | 100.4 | 100.1 | 98.5 | 99.7 | 100.0 |

**Table 6 Accelerated Test Results for Capsule Formulation (Example 2)**

| Evaluation Parameter | Requirements | Starting time | 1 Month | 2 Months | 3 Months | 6 Months | |
|---|---|---|---|---|---|---|---|
| Appearance | Content should be pale yellow to yellow powder or granules | Yellow Granules | Yellow Granules | Yellow Granules | Yellow Granules | Yellow Granules | |
| Related Substances (%) | Individual Impurities | should be <0.5% | 0.04 | 0.04 | 0.04 | 0.04 | 0.04 |
| | Total Impurities | should be ≤1.5% | 0.23 | 0.30 | 0.26 | 0.31 | 0.42 |
| Dissolution (%) | | Should be ≥80% of labeled amount | 99.0 | 91.3 | 97.8 | 100.2 | 98.2 |
| Content (%) | | C₃₀H₃₂D₃N₇O₂ should be 90% ∼ 110% of labeled amount | 100.7 | 101.9 | 98.5 | 100.8 | 100.9 |

**Table 7 Long-Term Stability Test Results for Capsule Formulation (Example 1)**

| Evaluation Parameter | | Requirements | Starting time | 3 Months | 6 Months |
|---|---|---|---|---|---|
| Appearance | | Content should be pale yellow to yellow powder or granules | Yellow Granules | Yellow Granules | Yellow Granules |
| Related Substances (%) | Maximum Individual Impurity | should be <0.5% | 0.04 | 0.04 | 0.04 |
| | Total Impurities | should be ≤1.5% | 0.20 | 0.32 | 0.41 |
| Dissolution (%) | | Should be ≥80% of labeled amount | | 98.9 | 96.7 |
| Content (%) | | C₃₀H₃₂D₃N₇O₂ should be 90% ∼ 110% of labeled amount | | 100.9 | 100.0 |

**Table 8 Long-Term Stability Test Results for Capsule Formulation (Example 2)**

| Evaluation Parameter | | Requirements | Starting time | 3 Months | 6 Months |
|---|---|---|---|---|---|
| Appearance | | Content should be pale yellow to yellow powder or granules | Yellow Granules | Yellow Granules | Yellow Granules |
| Related Substances (%) | Maximum Individual Impurity | should be ≤0.5% | 0.04 | 0.03 | 0.04 |
| | Total Impurities | should be ≤1.5% | 0.23 | 0.26 | 0.32 |
| Dissolution (%) | Should be ≥80% of labeled amount | 99.0 | 100.4 | 97.7 | |
| Microbiological Limits | Should comply with regulations | Complies with regulations | - | Complies with regulations | |
| Content (%) | C₃₀H₃₂D₃N₇O₂ should be 90% ∼ 110% of labeled amount | 100.7 | 101.3 | 100.7 | |

Accelerated and long-term stability tests were conducted on products derived from both Example 3 and Example 4. These products were enclosed within double aluminum blister packaging and subjected to a 3-month accelerated test followed by a 3-month long-term stability test. They were stored at a controlled temperature of 30°C±2°C and a relative humidity of 65%±5%. The results of these tests have been documented in Table 9, Table 10, Table 11, and Table 12. The findings conclusively demonstrate that throughout both the 3-month accelerated testing and the subsequent 3-month long-term stability tests, all parameters met the specified requirements. This further confirms the efficacy of the packaging in safeguarding the quality of the medication.

**Table 9 Accelerated and Stability Test Results for Capsule Formulation (Example 3)**

| Evaluation Parameter | | Requirements | Starting time | 1 Month | 2 Months | 3 Months |
|---|---|---|---|---|---|---|
| Appearance | | Content should be pale yellow to yellow powder or granules | Yellow Granules | Yellow Granules | Yellow Granules | Yellow Granules |
| Related Substances (%) | Maximum Individual Impurity | should be <0.5% | 0.10 | 0.09 | 0.11 | 0.10 |
| | Total Impurities | should be ≤1.5% | 0.61 | 0.56 | 0.69 | 0.64 |
| Dissolution (%) | | Should be ≥80% of labeled amount | 97.7 | 102.9 | 101.2 | 100.0 |
| Content (%) | | C₃₀H₃₂D₃N₇O₂ should be 90% ∼ 110% of labeled amount | 97.7 | 98.0 | 97.5 | 97.5 |

**Table 10 Accelerated Test Results for Capsule Formulation (Example 4)**

| Evaluation Parameter | | Requirements | Starting time | 1 Month | 2 Month | 3 Month |
|---|---|---|---|---|---|---|
| Appearance | | Content should be pale yellow to yellow powder or granules | Yellow Granules | Yellow Granules | Yellow Granules | Yellow Granules |
| Related Substances (%) | Maximum Individual Impurity | should be <0.5% | 0.09 | 0.09 | 0.10 | 0.10 |
| | Total Impurities | should be ≤1.5% | 0.60 | 0.55 | 0.63 | 0.68 |
| Dissolution (%) | | Should be ≥80% of labeled amount | 102.8 | 98.3 | 104.2 | 101.8 |
| Content (%) | | C₃₀H₃₂D₃N₇O₂ should be 90% ∼ 110% of labeled amount | 103.1 | 103.7 | 103.7 | 103.1 |

**Table 11 Long-Term Stability Test Results for Capsule Formulation (Example 3)**

| Evaluation Parameter | | Requirements | Starting time | 3 Months |
|---|---|---|---|---|
| Appearance | | Content should be pale yellow to yellow powder or granules | Yellow Granules | Yellow Granules |
| Related Substances (%) | Maximum Individual Impurity | should be <0.5% | 0.10 | 0.09 |
| | Total Impurities | should be ≤1.5% | 0.61 | 0.63 |
| Dissolution (%) | | Should be ≥80% of labeled amount | Should be ≥80% of labeled amount | 99.9 |
| Content (%) | | C₃₀H₃₂D₃N₇O₂ should be 90% ∼ 110% of labeled amount | C₃₀H₃₂D₃N₇O₂, should be 90% ∼ 110% of labeled amount | 98.2 |

**Table 12 Long-Term Stability Test Results for Capsule Formulation (Example 4)**

| Evaluation Parameter | | Requirements | Starting time | 3 Months |
|---|---|---|---|---|
| Appearance | | Content should be pale yellow to yellow powder or granules | Yellow Granules | Yellow Granules |
| Related Substances (%) | Maximum Individual Impurity | should be <0.5% | 0.09 | 0.10 |
| | Total Impurities | should be ≤1.5% | 0.60 | 0.66 |
| Dissolution (%) | | Should be ≥80% of labeled amount | 102.8 | 102.4 |
| Content (%) | | C₃₀H₃₂D₃N₇O₂ should be 90% ∼ 110% of labeled amount | 103.1 | 104.1 |

Stress testing was carried out on products derived from both Example 3 and Example 4, as outlined in Table 13 and Table 14. The results indicate that, when subjected to high temperature and light exposure, all testing parameters met the specified requirements without significant changes. However, when exposed to high humidity conditions (RH 92.5%) for a period of 10 days, moisture absorption increased by more than 5%. In contrast, under conditions of high humidity (RH 75%), moisture absorption increased by less than 5%, and all other testing parameters remained within acceptable limits without notable deviations. These findings suggest that the product is sensitive to moisture and should be stored in dry conditions. Considering the potential impact of microorganisms, it is advisable to keep the product sealed.

Furthermore, stress testing was conducted on samples with packaging, and the results are summarized as follows: under light exposure, all testing parameters consistently met the specified requirements without any significant alterations. When exposed to high humidity (RH 92.5%) for a duration of 10 days, the samples exhibited minimal moisture absorption. Additionally, all other testing parameters remained in compliance without notable variations. This demonstrates the effective moisture barrier properties of the product's packaging.

**Table 13 - Stress Testing Results (Example 3)**

| Testing Aspect | Requiremen ts | Starting time | High Humidity (RH75%, 10 days) (Unpacka ged) | High Humidity (RH92.5%, 10 days) (Packaged) | Light Exposure (12days) (Unpacka ged) | Light Exposure (12days) (Packaged ) | High Temperat ure 60°C, 30 days (Unpacka ged) | |
|---|---|---|---|---|---|---|---|---|
| Appearance | After capsule removal: Light yellow to yellow powder/granules | Yellow Granules | Yellow Granules | Yellow Granules | Yellow Granules | Yellow Granules | Yellow Granules | |
| Relat ed Subst ances (%) | Maxim um Individ ual Impurit y | should be ≤0.5% | 0.10 | 0.07 | 0.07 | 0.07 | 0.08 | 0.08 |
| | Total Impuriti es | should be ≤1.5% | 0.61 | 0.64 | 0.63 | 0.66 | 0.68 | 0.74 |
| Dissolution (%) | | ≥80% of labeled amount | 97.7 | 98.7 | 97.1 | 99.2 | 98.0 | 94.7 |
| Content (%) | | 90% ∼ 110% of labeled amount | 97.7 | 100.7 | 100.7 | 98.5 | 97.9 | 95.3 |

**Table 14 - Stress Testing Results (Example 4)**

| Testing Aspect | | Requiremen ts | Starting time | High Humidity (RH75%, 10 days) (Unpacka ged) | High Humidity (RH92.5%, 10 days) (Packaged) | Light Exposure (12days) (Unpacka ged) | Light Exposure (12days) (Packaged ) | High Temperat ure 60°C, 30 days (Unpacka ged) |
|---|---|---|---|---|---|---|---|---|
| Appearance | | After capsule removal: Light yellow to yellow powder/gra nules | Yellow Granules | Yellow Granules | Yellow Granules | Yellow Granules | Yellow Granules | Yellow Granules |
| Relat ed Subst ances (%) | Maxim um Individ ual Impurit y | should be ≤0.5% | 0.09 | 0.07 | 0.08 | 0.08 | 0.08 | 0.10 |
| | Total Impuriti es | should be ≤1.5% | 0.60 | 0.63 | 0.66 | 0.68 | 0.65 | 0.79 |
| Moisture Absorption | | ≤5% | - | 3.6 | 0.1 | - | - | - |
| Dissolution (%) | ≥80% of labeled amount | 102.8 | 101.8 | 104.5 | 102.7 | 102.8 | 102.0 | |
| Content (%) | 90% ∼ 110% of labeled amount | 103.1 | 104.1 | 105.0 | 103.5 | 103.9 | 102.6 | |

## Claims

1. Pharmaceutical formulations comprising N-(5-((4-(5,6-dihydro-4H-pyrrolo [3,2,1-ij]quinolin-1-yl)piperidin-2-yl)amino)-4-methoxy-2-(methyl(2-(methyl-d₃) amino)ethyl)phenyl)acetamide or a pharmaceutically acceptable salt thereof as active ingredient, along with at least one pharmaceutically acceptable excipient, formulated as an orally administrable capsule dosage form; preferably, the active ingredient is N-(5-((4-(5,6-dihydro-4H-pyrrolo[3,2,1-ij]quinolin-1-yl)piperidin -2-yl)amino)-4-methoxy-2-(methyl(2-(methyl-d₃)amino)ethyl)phenyl)acetamide methanesulfonate.

2. The pharmaceutical formulations of claim 1, **characterized by** encompassing the following components, or consisting of the following components:
| | |
|---|---|
| Active Ingredient: | 1-40% |
| Filler: | 1-80% |
| Disintegrant: | 0-10% |
| Lubricant: | 1-5% |
The percentages of each component are in accordance with weight percentages, and the sum of the percentages of all components totals 100%;
Preferably, it encompasses the following components or consists of the following components:
| | |
|---|---|
| Active Ingredient: | 20-38% |
| Filler: | 60-75% |
| Lubricant: | 1-5% |
The percentages of each component are in accordance with weight percentages, and the sum of the percentages of all components totals 100%.

3. The pharmaceutical formulations of claim 2, where the filler is selected from microcrystalline cellulose, mannitol, or lactose, either individually or in combination; preferably, the filler is microcrystalline cellulose or mannitol, either individually or in combination.

4. The pharmaceutical formulations of claim 2, where the disintegrant is selected from sodium starch glycolate, cross-linked sodium carboxymethylcellulose, or low-substituted hydroxypropyl cellulose, preferably, it is low-substituted hydroxypropyl cellulose; the lubricant is selected from talc, magnesium stearate, colloidal silicon dioxide, or sodium stearyl fumarate, either individually or in combination, preferably, it is a sodium stearyl fumarate, magnesium stearate, or talc, either individually or in combination.

5. The pharmaceutical formulations of claim 2, including the following components, or consisting of the following components:
| | |
|---|---|
| Active Ingredient: | 23.60-37.82% |
| Microcrystalline Cellulose: | 60.60-74.72% |
| Magnesium Stearate: | 0.50-0.52% |
| Talc: | 1.08-1.12% |
The percentages of each component are in accordance with weight percentages, and the sum of the percentages of all components totals 100%.

6. A pharmaceutical formulation comprising the following components by weight percentage:
| | |
|---|---|
| Active Ingredient: | 8.43% |
| Mannitol: | 25.36% |
| Microcrystalline Cellulose: | 59.21% |
| Low-Substituted Hydroxypropyl Cellulose: | 5.00% |
| Magnesium Stearate: | 0.50% |
| Talc: | 1.50% |
The active ingredient is N-(5-((4-(5,6-dihydro-4H-pyrrolo[3,2,1-ij] quinolin-1-yl)piperidin-2-yl)amino)-4-methoxy-2-(methyl(2-(methyl-d₃)amino)et hyl)phenyl)acetamide or the pharmaceutically acceptable salt thereof; preferably, the active ingredient is N-(5-((4-(5,6-dihydro-4H-pyrrolo[3,2,1-ij]quinolin-1-yl) piperidin-2-yl)amino)-4-methoxy-2-(methyl(2-(methyl-d₃)amino)ethyl)phenyl)ac etamide methanesulfonate.

7. A pharmaceutical formulation comprising the following components by weight percentage:
| | |
|---|---|
| Active Ingredient: | 23.65% |
| Microcrystalline Cellulose: | 69.35% |
| Low-Substituted Hydroxypropyl Cellulose: | 5.00% |
| Magnesium Stearate: | 0.50% |
| Talc: | 1.50% |
The active ingredient is N-(5-((4-(5,6-dihydro-4H-pyrrolo[3,2,1-ij] quinolin-1-yl)piperidin-2-yl)amino)-4-methoxy-2-(methyl(2-(methyl-d₃)amino)et hyl)phenyl)acetamide or the pharmaceutically acceptable salt thereof; preferably, the active ingredient is N-(5-((4-(5,6-dihydro-4H-pyrrolo[3,2,1-ij] quinolin-1-yl)piperidin-2-yl)amino)-4-methoxy-2-(methyl(2-(methyl-d₃)amino)et hyl)phenyl)acetamide methanesulfonate.

8. A pharmaceutical formulation comprising the following components by weight percentage:
| | |
|---|---|
| Active ingredient: | 23.64% |
| Microcrystalline cellulose: | 74.72% |
| Magnesium stearate: | 0.52% |
| Talc: | 1.12% |
The active ingredient is N-(5-((4-(5,6-dihydro-4H-pyrrolo[3,2,1-ij]quinolin-1-yl) pyrimidin-2-yl)amino)-4-methoxy-2-(methyl(2-(methyl(methyl-d₃)amino)ethyl)a mino)phenyl)acetamide or its pharmaceutically acceptable salt; preferably, the active ingredient is N-(5-((4-(5,6-dihydro-4H-pyrrolo[3,2,1-ij]quinolin-1-yl) pyrimidin-2-yl)amino)-4-methoxy-2-(methyl(2-(methyl(methyl-d₃)amino)ethyl)a mino)phenyl)acetamide methanesulfonate.

9. A pharmaceutical formulation, **characterized by** comprising the following weight percentages of components:
| | |
|---|---|
| Active ingredient: | 37.82% |
| Microcrystalline cellulose: | 60.60% |
| Magnesium stearate: | 0.50% |
| Talc: | 1.08% |
The active ingredient is N-(5-((4-(5,6-dihydro-4H-pyrrolo[3,2,1-ij] quinolin-1-yl)pyrimidin-2-yl)amino)-4-methoxy-2-(methyl(2-(methyl(methyl-d₃) amino)ethyl)amino)phenyl)acetamide or its pharmaceutically acceptable salt; preferably, the active ingredient is N-(5-((4-(5,6-dihydro-4H-pyrrolo[3,2,1-ij] quinolin-1-yl)pyrimidin-2-yl)amino)-4-methoxy-2-(methyl(2-(methyl(methyl-d₃) amino)ethyl)amino)phenyl)acetamide methanesulfonate.

10. A preparation method for any one of the pharmaceutical formulations according to claim 2 to claim 9, **characterized by** including the following steps:
(1) Mixing: After sieving the raw materials, take the active ingredient and a portion of the filler as specified in the pharmaceutical formulations and mix it with at 10-20 rpm for 4-10 min; add the remaining filler and continue mixing at 10-20 rpm for 4-10 min; add the lubricant and mix at 10-20 rpm for 8-15 min;
(2) Capsule filling: At the temperature of 15°C to 25°C and relative humidity between 45% and 65%, calculate the actual filling quantity by considering the intermediate content and theoretical filling content, and proceed with capsule filling process;
Preferably, 40% to 60% of the filler from the pharmaceutical formulation as in step (1), more preferably, half the weight of the filler is required in the pharmaceutical formulation.
